# FASCICULE DE BREVET EUROPEEN

(11) **EP 2 999 951 B1**
(45) Date de publication et mention de la délivrance du brevet: **19.06.2019**
(21) Numéro de dépôt: 14731917.2
(22) Date de dépôt: 21.05.2014
(51) Int. Cl.: G01M 3/26, A61F 2/00

(54) **PROCEDE ET DISPOSITIF DE DETECTION D'UNE FUITE LENTE DANS UN SYSTEME OCCLUSIF HYDRAULIQUE IMPLANTABLE**
VERFAHREN UND VORRICHTUNG ZUM DETEKTIEREN EINES LANGSAMEN LECKS IN EINEM IMPLANTIERBAREN HYDRAULISCHEN OKKLUSIVEN SYSTEM
METHOD AND DEVICE FOR DETECTING A SLOW LEAK IN A IMPLANTABLE HYDRAULIC BANDING SYSTEM

(30) Priorité: 21.05.2013 FR 1354538
(43) Date de publication de la demande: 30.03.2016
(73) Titulaire: Uromems, 38000 Grenoble (FR)
(72) Inventeur: LAMRAOUI, Hamid, F-38000 Grenoble (FR)
(74) Mandataire: Regimbeau
(86) Numéro de dépôt international: PCT/EP2014/060468
(87) Numéro de publication internationale: WO 2014/187871

(56) Documents cités:
- US-A1- 2010 152 532
- US-A1- 2012 265 456

## Description

### DOMAINE DE L'INVENTION

La présente invention concerne un procédé et un dispositif de détection de fuites lentes dans un système hydraulique d'occlusion implantable dans le corps humain ou animal pour occlure un conduit naturel.

Cette invention s'applique à tout type de système d'occlusion, incluant les sphincters artificiels urinaire, anal, oesophagien ou pylorique ou encore les anneaux gastriques.

### ARRIERE PLAN DE L'INVENTION

L'implantation de systèmes occlusifs pour occlure totalement ou partiellement un conduit naturel d'un patient est connue pour différentes indications.

Par exemple, le traitement de l'incontinence urinaire peut impliquer l'implantation, chez un patient, d'un sphincter artificiel.

Un tel sphincter comprend typiquement un élément occlusif placé autour de l'urètre (chez l'homme ou la femme), parfois du col vésical (chez la femme) ou de la prostate (chez l'homme) dans le but d'exercer une compression directe ou indirecte sur l'urètre afin d'éviter des fuites urinaires, un dispositif d'actionnement dudit élément occlusif pour faire varier la compression exercée sur l'urètre ou le col vésical, ainsi qu'une unité de commande du dispositif d'actionnement.

Dans le cas d'un système hydraulique, l'élément occlusif est une manchette gonflable contenant un volume variable d'un fluide et le dispositif d'actionnement comprend un réservoir contenant un fluide en liaison fluidique avec la manchette et un actionneur permettant d'ajouter ou de retirer ledit fluide pour compresser ou décompresser la manchette.

Un tel sphincter artificiel est décrit en particulier dans [1] et [2].

Un autre exemple de sphincter artificiel est décrit dans [3].

Dans un tel système, il est possible que des fuites lentes se produisent dans le circuit hydraulique.

Par « fuite lente », on entend dans le présent texte une perte (respectivement un apport) de liquide en petite quantité et s'étalant sur une longue plage de temps (au moins plusieurs jours, mais plus généralement de l'ordre de plusieurs mois), occasionnant une diminution (respectivement une augmentation) progressive de la pression dans le circuit hydraulique.

Par opposition, une fuite est considérée comme rapide si elle cause une diminution brutale de la pression dans le circuit hydraulique.

Ainsi, une fuite rapide est détectable dès que l'événement déclencheur a eu lieu (par exemple, une déconnexion de la tubulure reliant la manchette au réservoir de fluide) ou peu après (par exemple, dans le cas d'un endommagement important de l'un des matériaux assurant l'étanchéité).

Les documents US 2010/0152532 et US 2012/0265456 décrivent des procédés de détection d'une fuite rapide dans une bande gastrique.

Au contraire, une fuite lente ne devient détectable que plusieurs jours, voire plusieurs mois après la survenance de la défaillance qui en est la cause.

De telles fuites lentes peuvent avoir différentes causes, parmi lesquelles :
- l'endommagement d'un élément mécanique du circuit hydraulique, par exemple une fissuration d'un élément,
- un défaut de la connexion de la tubulure au réservoir ou à la manchette occlusive,
- la porosité du matériau constituant la tubulure et/ou la manchette occlusive (qui sont généralement en silicone) et une concentration inadéquate de la solution saline contenue dans le circuit hydraulique, générant un gradient de concentration entre le milieu extracellulaire et le circuit hydraulique qui entraîne une diffusion d'eau vers le milieu le plus concentré. Ainsi, si le fluide contenu dans le circuit hydraulique est trop peu concentré (hypotonique) par rapport au milieu extérieur, l'eau tendra à diffuser vers l'extérieur du circuit hydraulique, conduisant à une diminution du volume dans ledit circuit ; au contraire, si le fluide contenu dans le circuit hydraulique est trop concentré (hypertonique), il se produira une diffusion d'eau provenant de l'extérieur vers le circuit hydraulique, conduisant à une augmentation du volume [4].

Une fuite lente a pour effet d'altérer le fonctionnement du système occlusif.

En effet, pour une même consigne d'actionnement, le fait que le circuit hydraulique contienne moins de fluide ou davantage que lors de l'implantation du système occlusif a pour effet de faire varier la compression exercée sur le conduit à occlure.

Dans le cas où la fuite lente se traduit par une perte de fluide, elle peut impliquer une diminution de l'occlusion exercée par la manchette, et par conséquent une diminution de l'efficacité du système occlusif.

Dans le cas où la fuite lente se traduit par un apport de fluide, elle implique une augmentation progressive de la pression dans le circuit hydraulique, pouvant occasionner une compression excessive des tissus entourés par la manchette.

Les systèmes occlusifs proposés actuellement ne permettent pas de détecter une fuite lente dans le circuit hydraulique ni d'en alerter le patient ou le praticien.

Un but de l'invention est donc de concevoir un procédé et un dispositif permettant de détecter ce type de fuite et, le cas échéant, alerter le praticien ou le patient.

### BREVE DESCRIPTION DE L'INVENTION

Conformément à l'invention, il est proposé un procédé de détection d'une fuite lente dans un système hydraulique d'occlusion implantable dans le corps humain ou animal pour occlure un conduit naturel, ledit système occlusif comprenant :
- un circuit hydraulique comprenant :
   - une manchette occlusive gonflable contenant un volume variable d'un fluide, entourant une partie du conduit naturel à occlure,
   - un réservoir contenant un fluide, et
   - une liaison fluidique entre la manchette et le réservoir,
- un dispositif d'actionnement couplé à un élément mobile dudit circuit hydraulique et adapté pour déplacer ledit élément mobile pour transférer un volume déterminé de fluide du réservoir vers la manchette ou de la manchette vers le réservoir, de sorte à faire varier la compression exercée par ladite manchette sur ledit conduit,
- une unité de commande adaptée pour solliciter le dispositif d'actionnement de sorte à exercer une compression déterminée sur le conduit,
la variation de la compression exercée par la manchette sur le conduit étant réalisée par transfert d'un volume ajustable dudit fluide entre le réservoir et la manchette.

Ledit procédé de détection comprend :
- la mesure de l'évolution de la pression dans le circuit hydraulique pour une sollicitation déterminée du dispositif d'actionnement, ladite sollicitation déterminée étant une position de l'élément mobile définissant un volume déterminé de fluide transféré,
- la détection d'une fuite lente dans le circuit hydraulique lorsque la pression mesurée dans ledit circuit pour ladite sollicitation déterminée du dispositif d'actionnement remplit un critère prédéterminé.

Dans le cadre de la présente invention, une fuite lente peut impliquer tout phénomène de transfert de fluide de ou vers le circuit hydraulique, mettant en oeuvre des mécanismes physiques et/ou chimiques, qu'il s'agisse d'un écoulement direct, d'une diffusion (notamment de type osmose), etc.

Par ailleurs, une telle fuite peut comprendre, le plus souvent, un transfert de fluide du circuit hydraulique vers le milieu extérieur, mais aussi, dans certains cas, du milieu extérieur vers le circuit hydraulique.

Selon une forme d'exécution de l'invention, ledit critère de détection prédéterminé peut être choisi parmi l'une des conditions suivantes, ou une combinaison desdites conditions :
- la pression dans le circuit hydraulique est inférieure à une valeur fixe,
- la pression dans le circuit hydraulique est inférieure à un pourcentage d'une valeur de la pression mesurée initialement pour ladite sollicitation déterminée du dispositif d'actionnement, et
- une valeur issue d'une fonction mathématique construite à partir d'une base de données de valeurs de ladite pression pour ladite sollicitation déterminée du dispositif d'actionnement, ladite pression étant enregistrée périodiquement au cours du temps, est inférieure à une valeur déterminée.

Dans ce cas, on détecte une fuite lente correspondant à une perte de fluide du circuit hydraulique.

Selon un mode de réalisation, le réservoir présente un volume variable, le volume étant ajusté par un déplacement linéaire d'un élément mobile mû par un actionneur.

Dans ce cas, la sollicitation déterminée du dispositif d'actionnement peut être une position de l'élément mobile en butée de sorte à maximiser le volume du réservoir.

Selon un mode d'exécution de l'invention, le critère de détection déterminé est rempli lorsque la pression mesurée dans le circuit hydraulique pour ladite sollicitation déterminée devient inférieure à une valeur seuil nulle ou négative.

Selon une autre forme d'exécution de l'invention, pour détecter une fuite lente se traduisant par un apport de fluide dans le circuit hydraulique, ledit critère de détection prédéterminé est choisi parmi l'une des conditions suivantes ou une combinaison desdites conditions :
- la pression dans le circuit hydraulique est supérieure à une valeur fixe,
- la pression dans le circuit hydraulique est supérieure à un pourcentage d'une valeur de la pression mesurée initialement pour ladite sollicitation déterminée du dispositif d'actionnement, et
- une valeur issue d'une fonction mathématique construite à partir d'une base de données de valeurs de ladite pression pour ladite sollicitation déterminée du dispositif d'actionnement, ladite pression étant enregistrée périodiquement au cours du temps, est supérieure à une valeur déterminée.

De manière particulièrement avantageuse, le procédé comprend en outre l'émission d'une alarme vers un utilisateur si le critère de détection d'une fuite lente est rempli.

Selon un mode de réalisation, le dispositif d'actionnement comprend une pompe péristaltique, le volume de fluide à transférer étant ajusté par un déplacement angulaire du rotor de ladite pompe.

Selon une application préférée mais non limitative de l'invention, le système occlusif est un sphincter urinaire artificiel.

Un autre objet concerne un dispositif permettant la mise en oeuvre dudit procédé.

Il s'agit plus précisément d'un dispositif de détection d'une fuite lente dans un système occlusif hydraulique implantable dans le corps humain ou animal pour occlure un conduit naturel, ledit système occlusif comprenant :
- un circuit hydraulique comprenant :
   - une manchette occlusive gonflable contenant un volume variable d'un fluide, entourant une partie du conduit naturel à occlure,
   - un réservoir contenant un fluide, et
   - une liaison fluidique entre la manchette et le réservoir,
- un dispositif d'actionnement couplé à un élément mobile dudit circuit hydraulique et adapté pour déplacer ledit élément mobile pour transférer un volume déterminé de fluide du réservoir vers la manchette ou de la manchette vers le réservoir, de sorte à faire varier la compression exercée par ladite manchette sur ledit conduit,
- une unité de commande adaptée pour solliciter le dispositif d'actionnement de sorte à exercer une compression déterminée sur le conduit.

Conformément à l'invention, ledit dispositif de détection comprend :
- un capteur adapté pour mesurer la pression dans le circuit hydraulique,
- un capteur adapté pour mesurer une sollicitation appliquée au dispositif d'actionnement,
- une unité de traitement adaptée pour :
   - à partir des données de mesure desdits capteurs, mesurer l'évolution de la pression dans le circuit hydraulique pour une sollicitation déterminée du dispositif d'actionnement, ladite sollicitation déterminée étant une position de l'élément mobile définissant un volume déterminé de fluide transféré,
   - détecter une fuite lente dans le circuit hydraulique lorsque la pression mesurée dans ledit circuit pour ladite sollicitation déterminée du dispositif d'actionnement remplit un critère déterminé.

Enfin, l'invention concerne un système occlusif hydraulique implantable dans le corps humain ou animal pour occlure un conduit naturel, comprenant un dispositif adapté pour mettre en oeuvre le procédé de détection d'une fuite lente tel que décrit ci-dessus.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma de principe d'un système occlusif hydraulique selon un mode de réalisation de l'invention,
- la figure 2 est un schéma de principe d'un système occlusif hydraulique selon un autre mode de réalisation de l'invention,
- la figure 3 est un schéma de principe de l'architecture de l'unité de commande d'un système occlusif incorporant un dispositif de détection d'une fuite lente,
- la figure 4A illustre un exemple de courbe de variation de la pression dans le circuit hydraulique en fonction du temps dans le cas d'une fuite lente ; la figure 4B présente les différentes positions du piston dans le cas d'un système occlusif hydraulique.

### DESCRIPTION DETAILLEE DE L'INVENTION

D'une manière générale, le système occlusif comprend un élément occlusif entourant un conduit naturel à occlure.

Selon l'application du système occlusif considéré, le conduit à occlure peut être un conduit urinaire (notamment l'urètre ou le col vésical), anal, oesophagien, pylorique ou encore l'estomac (cas d'un anneau gastrique).

L'occlusion dudit conduit peut être totale (cas d'un sphincter urinaire destiné à éviter des fuites urinaires) ou partielle (cas d'un anneau gastrique destiné à limiter le passage des aliments dans l'estomac).

Le sphincter artificiel comprend également un dispositif d'actionnement pour ajuster la compression exercée par l'élément occlusif.

Il existe donc une liaison entre l'élément occlusif et le dispositif d'actionnement, qui dépend du mode d'actionnement dudit élément occlusif.

S'agissant d'un système occlusif hydraulique, l'élément occlusif est une manchette gonflable susceptible de contenir un volume ajustable d'un fluide et le dispositif d'actionnement comprend un réservoir de fluide, la liaison entre la manchette et le dispositif d'actionnement comprenant une tubulure permettant de transférer du fluide de manière bidirectionnelle de la manchette au réservoir selon que l'on souhaite augmenter ou diminuer la compression exercée.

Dans les systèmes occlusifs les plus anciens, ce dispositif d'actionnement est commandé manuellement par le patient, par exemple par une pression exercée sur un dispositif de pompe agencé sous la peau.

A l'heure actuelle, des systèmes plus perfectionnés sont développés afin d'éviter au patient d'exercer une pression manuelle sur la pompe pour contrôler l'élément occlusif.

Le système occlusif comprend alors une unité de commande, également implantable dans le corps du patient, qui est adaptée pour commander le dispositif d'actionnement de la manchette.

Il existe actuellement différents types de systèmes occlusifs hydrauliques, employant différentes technologies d'actionnement.

Ces différents systèmes occlusifs sont connus de l'homme du métier.

S'agissant des sphincters urinaires artificiels, on pourra par exemple se référer aux documents suivants [1] à [3] cités plus haut.

Le dispositif d'actionnement et l'unité de commande sont avantageusement inclus dans un boîtier implantable dans le corps du patient destiné à les protéger.

Le boîtier est typiquement réalisé en un matériau biocompatible.

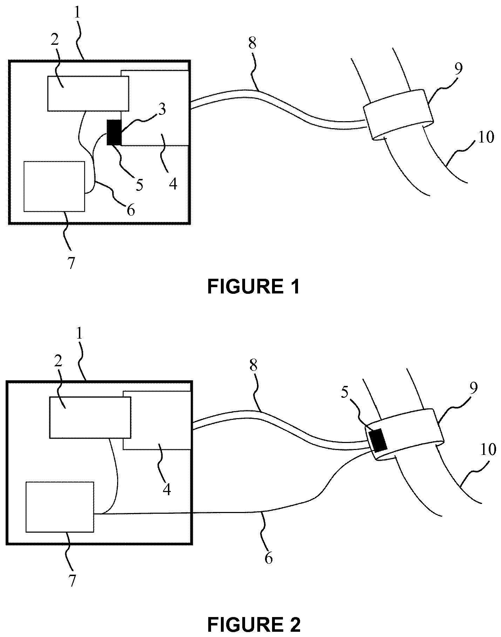

La figure 1 est un schéma de principe d'un système occlusif hydraulique associé à un dispositif de détection de fuite selon un mode de réalisation de l'invention.

L'élément occlusif 9 se présente sous la forme d'une manchette gonflable susceptible d'être remplie d'une quantité variable d'un fluide, une variation de la pression de fluide à l'intérieur de la manchette faisant varier la compression exercée sur le conduit naturel 10 à occlure.

Un réservoir 4 d'un fluide, par exemple du sérum physiologique, est agencé en liaison fluidique avec la manchette, par l'intermédiaire d'une tubulure 8.

L'ensemble de la manchette 9, du réservoir 4 et de la tubulure 2 forme le circuit hydraulique du système occlusif.

Ce circuit hydraulique permet de transférer une partie du fluide du réservoir dans la manchette, afin d'augmenter la compression exercée sur le conduit 10, et inversement de transférer une partie du fluide de la manchette vers le réservoir, pour diminuer la compression exercée par la manchette sur le conduit 10.

A cet effet, le système occlusif comprend en outre un dispositif d'actionnement 2 qui est couplé au circuit hydraulique pour effectuer ce transfert de fluide et ainsi faire varier la compression exercée par la manchette sur le conduit 10.

Ce transfert de fluide implique un déplacement d'un élément mobile par rapport à un élément fixe. Selon la forme d'exécution retenue, ce déplacement peut être une translation ou une rotation.

Selon un mode de réalisation avantageux, le réservoir 4 présente un volume variable.

Par exemple, mais de manière non limitative, la variation de volume peut être réalisée en déplaçant linéairement une paroi du réservoir, le dispositif d'actionnement 2 comprenant un actionneur pour déplacer ladite paroi.

Ainsi, le réservoir peut comprendre une membrane roulante, un piston, un soufflet ou tout autre moyen permettant de faire varier son volume par un déplacement linéaire d'un élément mobile par rapport à un élément fixe formant le corps du réservoir.

L'homme du métier est en mesure de sélectionner parmi les actionneurs existants un actionneur adéquat en fonction de l'implémentation envisagée vis-à-vis du réservoir.

On peut citer par exemple mais de manière non limitative un actionneur piézo-électrique, etc.

Bien qu'il ne soit pas illustré ici, le dispositif d'actionnement comprend un capteur permettant de mesurer l'action exercée sur le réservoir.

Par exemple, si l'actionnement consiste en un déplacement d'une paroi mobile du réservoir, ledit capteur peut consister en un capteur de position, permettant de déterminer la position de la paroi mobile.

Un calibrage permet de déterminer d'une part, la relation entre la position de la paroi mobile et la variation de volume du réservoir ; d'autre part, la relation entre la variation de volume du réservoir et la pression dans le circuit hydraulique et enfin entre la pression dans le circuit hydraulique et la compression exercée sur le conduit à occlure.

La relation entre la pression dans le circuit hydraulique et le volume transféré du réservoir vers la manchette gonflable peut éventuellement être exprimée sous la forme d'une relation mathématique.

Selon les cas, cette relation peut être linéaire ou non.

Ainsi, il est possible de déterminer le déplacement à imposer à la paroi mobile pour obtenir une pression donnée de fluide dans le circuit hydraulique en vue d'obtenir une compression donnée du conduit 10.

Dans ce cas, le contrôle en déplacement du dispositif d'actionnement est basé sur une mesure de la pression dans le circuit hydraulique.

A cet effet, dans le mode de réalisation illustré sur la figure 1, un capteur de pression 5 est agencé sur une paroi du réservoir 4 de manière à fournir une mesure de la pression de fluide dans le réservoir.

Le système occlusif comprend en outre une unité de commande 7 adaptée pour solliciter le dispositif d'actionnement 2 de sorte à exercer une compression déterminée sur le conduit 10.

La sollicitation consiste en une action qui doit être exercée par le dispositif d'actionnement pour obtenir une compression déterminée du conduit.

La liaison 6 entre l'unité de commande 7 et le dispositif d'actionnement 2 a été représentée sous forme filaire sur la figure 1, mais il va de soi qu'elle pourrait être mise en oeuvre sans fil, selon la technologie sélectionnée par l'homme du métier.

Il existe également une liaison 6 (filaire ou non) entre le capteur 5 et l'unité de commande 7.

La figure 2 illustre un autre mode de réalisation d'un système occlusif hydraulique.

Les composants désignés par les mêmes signes de référence que sur la figure 1 remplissent la même fonction et ne seront donc pas décrits en détail à nouveau.

Par rapport au dispositif illustré sur la figure 1, le capteur 5 permettant de mesurer la pression dans le circuit hydraulique n'est pas agencé sur une paroi du réservoir 4 mais sur la manchette occlusive 9, afin de mesurer directement la pression sur le conduit 10 du fluide dans ladite manchette.

Il va de soi que l'on pourrait mettre en place un capteur de pression en tout autre endroit du circuit hydraulique ou du système sans pour autant sortir du cadre de la présente invention.

Dans un autre mode de réalisation (non illustré), le dispositif d'actionnement comprend une pompe péristaltique permettant de transférer un fluide contenu dans le réservoir vers la manchette occlusive et inversement. Une telle pompe présente typiquement une tête fixe, dans laquelle est maintenue une tubulure déformable, et un rotor mobile en rotation dans la tête, portant des galets qui déforment et obturent la tubulure déformable. La rotation du rotor et des galets provoque l'entraînement du volume de fluide contenu dans la tubulure entre deux galets adjacents et permet le transfert dudit volume du réservoir vers la manchette ou inversement.

Les modes de réalisation décrits ci-dessus ne sont pas destinés à limiter l'invention et l'on pourra choisir d'autres moyens d'actionnement et d'autres capteurs sans sortir de la portée de la présente invention.

### Mesure de la pression dans le circuit hydraulique

La mesure de la pression dans le circuit hydraulique peut être réalisée en tout point dudit circuit, par exemple avec un ou plusieurs des capteurs de pression mentionnés plus haut.

Dans la présente invention, on s'intéresse à l'évolution de la pression dans le circuit hydraulique dans une situation particulière, correspondant à une sollicitation déterminée du dispositif d'actionnement.

Cette sollicitation déterminée dépend du type de dispositif d'actionnement mis en oeuvre dans le système occlusif.

Dans la mesure où le transfert de fluide implique un déplacement (en translation ou en rotation) d'un élément mobile, la sollicitation est avantageusement une position dudit élément.

Par exemple, lorsque le réservoir comprend une paroi mobile permettant de faire varier son volume et que le dispositif d'actionnement est adapté pour déplacer ladite paroi d'une distance déterminée, la sollicitation déterminée dans laquelle on surveille la pression dans le circuit hydraulique peut correspondre à la position de la paroi définissant un volume déterminé de fluide dans le réservoir.

Selon une forme d'exécution préférée, on choisit pour ladite position une position de butée ouverte de ladite paroi (correspondant à un volume maximal du réservoir, la manchette occlusive étant alors vide).

Lorsqu'il se produit une fuite lente dans le circuit hydraulique, la pression dans le circuit hydraulique pour cette position déterminée de la paroi tend à diminuer, et peut même devenir négative.

De manière similaire, dans le cas où le dispositif d'actionnement comprend une pompe péristaltique, la sollicitation pour laquelle on surveille l'évolution de la pression dans le circuit hydraulique peut être définie pour une position angulaire de référence du rotor par rapport à la tête de ladite pompe permettant d'obtenir une compression donnée du conduit ou permettant de maximiser le volume du réservoir associé à la pompe péristaltique.

### Surveillance de la pression au cours du temps

Pour détecter une éventuelle fuite lente dans le circuit hydraulique, on enregistre périodiquement la pression dans le circuit hydraulique pour la sollicitation prédéterminée décrite ci-dessus.

La périodicité de mesure n'est pas nécessairement régulière, c'est-à-dire qu'il peut s'écouler des intervalles de temps de différentes longueurs entre deux mesures consécutives.

Par ailleurs, l'enregistrement de la pression n'est pas nécessairement effectué à chaque fois que la sollicitation prédéterminée est rencontrée, mais peut éventuellement être réalisé moins souvent, selon une fréquence déterminée par le praticien.

D'autre part, la périodicité de mesure peut dépendre du type de système occlusif considéré.

Par exemple, pour un système occlusif urinaire ou anal, on procède au moins une fois par jour à une ouverture de la manchette occlusive pour permettre la miction ou la défécation.

Par conséquent, pour ces systèmes, on peut surveiller la pression dans le circuit hydraulique quotidiennement, en enregistrant la pression dans le circuit hydraulique lors d'au moins une miction ou défécation.

Dans d'autres systèmes (par exemple les anneaux gastriques), le système peut se calibrer soit lors d'une commande effectuée par l'utilisateur, soit de manière autonome en plaçant le dispositif d'actionnement dans une position de référence, dans des conditions qui n'affecte pas la fonction du dispositif.

### Critère de détection d'une fuite lente

Le critère de détection d'une fuite lente peut être sélectionné parmi différentes possibilités, dont certaines seront décrites ci-dessous.

On s'intéresse dans un premier temps au cas où la fuite lente se traduit par une perte de fluide du circuit hydraulique.

Selon une première forme d'exécution de l'invention, le critère de détection d'une telle fuite lente est rempli lorsque la pression dans le circuit hydraulique devient inférieure à une valeur fixe, indépendante du patient, fixée arbitrairement.

Cette valeur seuil est de préférence nulle ou négative.

Selon une autre forme d'exécution, le critère de détection d'une fuite lente est rempli lorsque la pression dans le circuit hydraulique devient inférieure à un pourcentage de la valeur de la pression mesurée initialement.

Par exemple, on mesure la valeur de la pression dans le circuit hydraulique pour une sollicitation déterminée du dispositif d'actionnement après l'implantation du système occlusif chez le patient, et l'on choisit cette valeur comme référence.

On choisit également un pourcentage de cette valeur de référence comme étant la valeur en-deçà de laquelle on détecte une fuite lente.

Par exemple, ce pourcentage pourrait être de l'ordre de 20 %.

Cette valeur peut être également négative et proportionnelle à un ou plusieurs paramètres du système.

Un avantage de ce critère est qu'il permet de tenir compte de la situation individuelle du patient, puisqu'il est basé sur une mesure faite sur le patient à l'initialisation du procédé de détection.

Selon une autre forme d'exécution de l'invention, le critère de détection d'une fuite lente implique la comparaison, non de la pression mesurée elle-même, mais d'une fonction de ladite pression, avec une valeur déterminée.

Ainsi, cette valeur à comparer à ladite valeur déterminée peut être une valeur issue d'une fonction mathématique construite à partir d'une base de données de valeurs de la pression mesurées périodiquement au cours du temps.

Ainsi, on peut par exemple considérer la présence d'une fuite lente lorsque le produit x.P passe sous une valeur seuil, x étant un paramètre pouvant évoluer dans le temps afin de prendre en compte le vieillissement des matériaux du système occlusif, et P la pression mesurée dans le circuit hydraulique pour une sollicitation donnée.

Par exemple, cette valeur seuil peut en particulier être négative.

Il est par ailleurs envisageable de définir un critère de détection plus complexe, qui prenne en compte simultanément différentes conditions en combinant différents critères dont ceux notamment présentés dans les paragraphes précédents.

De manière avantageuse, le choix d'une valeur seuil nulle ou négative permet de distinguer une fuite lente occasionnant une perte de fluide et une atrophie du conduit naturel.

Une atrophie est un amincissement localisé des tissus comprimés par la manchette occlusive.

Par conséquent, elle se traduit par une variation de la sollicitation à appliquer au dispositif d'actionnement pour obtenir une compression déterminée du conduit.

Cependant, dans ce cas, la pression dans le circuit hydraulique reste toujours positive pour une sollicitation déterminée du dispositif d'actionnement.

L'observation d'une pression inférieure à une valeur seuil nulle ou négative dans le circuit hydraulique pour une sollicitation déterminée du dispositif d'actionnement est donc caractéristique d'une fuite lente.

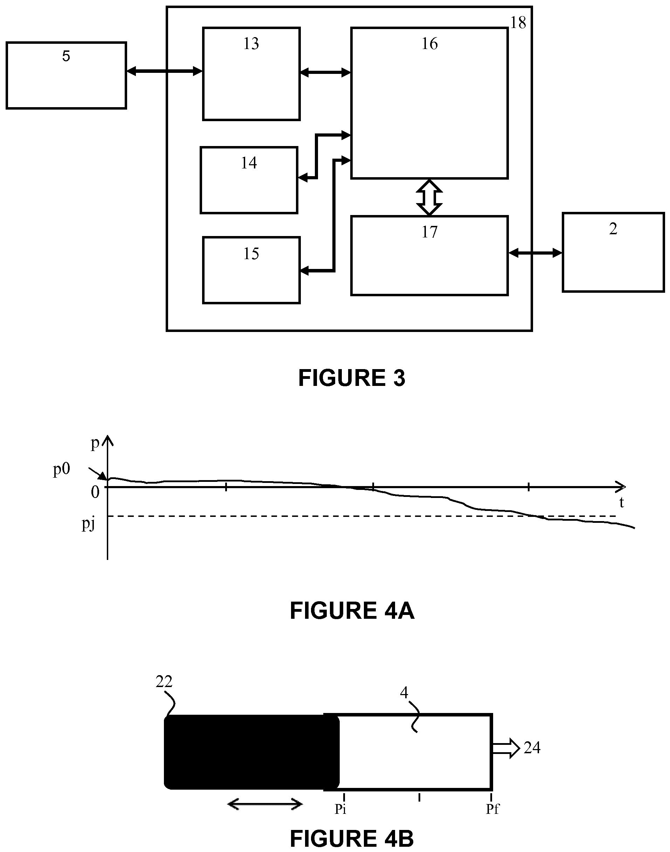

La figure 3 illustre une forme d'exécution de l'architecture générale de l'unité de traitement permettant la détection d'une éventuelle fuite lente.

L'unité de traitement 18 comprend un microprocesseur 16 adapté pour implémenter un algorithme permettant de commander une mesure de la pression lorsque la sollicitation prédéterminée du dispositif d'actionnement est observée, de traiter la mesure et pour comparer la pression mesurée à au moins un critère de détection d'une fuite lente.

A cet effet, le microprocesseur 16 communique avec au moins un capteur de pression 5 au moyen d'une interface 13.

Avantageusement, le microprocesseur communique également avec un capteur du dispositif d'actionnement, représentatif de la sollicitation exercée sur le dispositif d'actionnement, permettant de déterminer si la sollicitation prédéterminée est appliquée.

La communication est schématisée par des flèches et peut être réalisée par une liaison filaire ou par une liaison sans fil, selon des protocoles connus.

L'unité de traitement 18 comprend en outre une mémoire 14 dans laquelle sont enregistrés le programme de détection, les valeurs de pression mesurées, ainsi que les conditions à remplir pour qu'une fuite lente soit détectée.

L'unité de traitement comprend en outre une ou plusieurs horloges 15.

Le microprocesseur 16 est relié à l'interface 13, la mémoire 14 et l'horloge 15.

Le microprocesseur 16 communique en outre avec le dispositif d'actionnement 2 par l'intermédiaire d'une interface 17.

Les figures 4A et 4B illustrent un exemple de détection d'une fuite lente dans un système occlusif hydraulique.

Dans le mode de réalisation représenté sur la figure 4B, le réservoir 4 a un volume variable grâce au déplacement d'un piston 22.

La course du piston est définie par deux positions limites : la position Pi, correspondant à une butée initiale du piston dans laquelle la manchette occlusive est vide, et la position Pf, correspondant à une butée finale du piston dans laquelle la manchette occlusive est remplie de fluide de sorte à exercer une compression maximale du conduit.

La flèche 24 indique la direction de sortie du fluide du réservoir vers la manchette occlusive.

On s'intéresse à la variation de la pression dans le circuit hydraulique lorsque le piston est dans la position Pi.

La pression dans le circuit hydraulique à l'initialisation du système occlusif est notée p0.

Le graphe de la figure 4A présente l'évolution de la pression p au cours du temps lorsque le piston est dans la position Pi, dans le cas où une fuite lente se produit.

Comme on peut le voir sur ce graphe, la pression p décroît progressivement à partir de la valeur initiale p0.

Lorsque la courbe de la pression p atteint un seuil prédéterminé noté pj, on considère qu'une fuite lente est détectée.

Le cas échéant, une alerte est émise par l'unité de traitement à l'attention du patient et/ou du praticien.

Le praticien peut alors décider d'ajouter un nouveau volume de fluide dans le circuit hydraulique, ce qui permet d'éviter une nouvelle intervention chirurgicale.

Cet ajout peut être fait au travers d'un port d'injection qui est généralement prévu sur le circuit hydraulique, en particulier sur le réservoir.

Le port d'injection peut comprendre un septum agencé dans l'une des parois du réservoir qui est placée en regard de la peau du patient, de manière à ce que le praticien puisse introduire, à travers la peau, une aiguille dans le réservoir.

Le septum est en un matériau biocompatible permettant d'assurer l'étanchéité du réservoir pendant et après le retrait de l'aiguille.

Le silicone est généralement employé pour cet usage.

### Cas où le fluide est hypertonique

Lorsque le fluide est une solution hypertonique, il existe un risque de diffusion de fluide de l'extérieur vers le circuit hydraulique, susceptible de provoquer une sur-pressurisation du circuit hydraulique [4].

L'invention permet de détecter une telle situation et d'alerter un utilisateur.

Dans ce cas, la pression pour une position de l'élément mobile du réservoir ou de la pompe péristaltique ou une sollicitation déterminée de l'actionneur va augmenter progressivement.

Le critère de détection peut être choisi parmi l'un des critères décrits ci-dessus avec la différence que la détection est effectuée lorsque la pression est supérieure à une valeur déterminée.

Lorsque la courbe de la pression atteint le seuil prédéterminé, on considère qu'une surpression est détectée.

Le cas échéant, une alerte est émise par l'unité de traitement à l'attention du patient et/ou du praticien.

Le praticien peut alors décider de retirer un volume de fluide dans le circuit hydraulique, ce qui permet d'éviter une compression trop importante des tissus.

Ce retrait peut être fait au travers d'un port d'injection qui est généralement prévu sur le circuit hydraulique, en particulier sur le réservoir.

### REFERENCES

[1] WO 2009/027196
[2] Development of a Novel Artificial Urinary Sphincter, H. Lamraoui et al, IEEE/ASME Transactions on Mechatronics, Vol. 15, No. 6, Dec. 2010
[3] US 6,162,238
[4] F. Maillet, J.-M. Buzelin, O. Bouchot, and G. Karam, "Management of artificial urinary sphincter dysfunction," European Urology, vol. 46, no. 2, pp. 241-246, Aug. 2004
[5] C. Hajivassiliou, "A review of the complications and results of implantation of the AMS artificial urinary sphincter," European Urology, vol. 35, no. 1, pp. 36-44, 1999

## Revendications

1. Procédé de détection d'une fuite lente dans un système occlusif hydraulique implantable dans le corps humain ou animal pour occlure un conduit naturel (10), ledit système occlusif comprenant :
- un circuit hydraulique comprenant :
- une manchette occlusive gonflable (9) contenant un volume variable d'un fluide, entourant une partie du conduit naturel (10) à occlure,
- un réservoir (4) contenant un fluide, et
- une liaison fluidique (8) entre la manchette (9) et le réservoir (4),
- un dispositif d'actionnement (2) couplé à un élément mobile (22) dudit circuit hydraulique et adapté pour déplacer ledit élément mobile pour transférer un volume déterminé de fluide du réservoir (4) vers la manchette (9) ou de la manchette vers le réservoir, de sorte à faire varier la compression exercée par ladite manchette (9) sur ledit conduit (10),
- une unité de commande (7) adaptée pour solliciter le dispositif d'actionnement (2) de sorte à exercer une compression déterminée sur le conduit (10),
la variation de la compression exercée par la manchette sur le conduit (10) étant réalisée par transfert d'un volume ajustable dudit fluide entre le réservoir (4) et la manchette (9),
ledit procédé comprenant :
- la mesure de l'évolution de la pression dans le circuit hydraulique pour une sollicitation déterminée du dispositif d'actionnement (2), ladite sollicitation déterminée étant une position de l'élément mobile (22) définissant un volume déterminé de fluide transféré,
- la détection d'une fuite lente dans le circuit hydraulique lorsque la pression mesurée dans ledit circuit pour ladite sollicitation déterminée du dispositif d'actionnement (2) remplit un critère prédéterminé.

2. Procédé selon la revendication 1, **caractérisé en ce que** pour détecter une fuite lente se traduisant par une perte de fluide du circuit hydraulique, ledit critère de détection prédéterminé est choisi parmi l'une des conditions suivantes ou une combinaison desdites conditions :
- la pression dans le circuit hydraulique est inférieure à une valeur fixe,
- la pression dans le circuit hydraulique est inférieure à un pourcentage d'une valeur de la pression mesurée initialement pour ladite sollicitation déterminée du dispositif d'actionnement, et
- une valeur issue d'une fonction mathématique construite à partir d'une base de données de valeurs de la pression dans le circuit hydraulique pour ladite sollicitation déterminée du dispositif d'actionnement, ladite pression étant enregistrée périodiquement au cours du temps, est inférieure à une valeur déterminée.

3. Procédé selon l'une des revendications 1 ou 2, **caractérisé en ce que** le réservoir (4) présente un volume variable, le volume étant ajusté par un déplacement linéaire d'un élément mobile (22) mû par un actionneur.

4. Procédé selon la revendication 3, **caractérisé en ce que** la sollicitation déterminée du dispositif d'actionnement (2) est une position de l'élément mobile (22) en butée de sorte à maximiser le volume du réservoir (4).

5. Procédé selon l'une des revendications 1 à 4, **caractérisé en ce que** le critère de détection déterminé est rempli lorsque la pression mesurée dans le circuit hydraulique pour ladite sollicitation déterminée devient inférieure à une valeur seuil nulle ou négative.

6. Procédé selon la revendication 1, **caractérisé en ce que** pour détecter une fuite lente se traduisant par un apport de fluide dans le circuit hydraulique, ledit critère de détection prédéterminé est choisi parmi l'une des conditions suivantes ou une combinaison desdites conditions :
- la pression dans le circuit hydraulique est supérieure à une valeur fixe,
- la pression dans le circuit hydraulique est supérieure à un pourcentage d'une valeur de la pression mesurée initialement pour ladite sollicitation déterminée du dispositif d'actionnement, et
- une valeur issue d'une fonction mathématique construite à partir d'une base de données de valeurs de ladite pression pour ladite sollicitation déterminée du dispositif d'actionnement, ladite pression étant enregistrée périodiquement au cours du temps, est supérieure à une valeur déterminée.

7. Procédé selon l'une des revendications 1 à 6, **caractérisé en ce qu'**il comprend en outre l'émission d'une alarme vers un utilisateur si le critère de détection d'une fuite lente est rempli.

8. Procédé selon l'une des revendications 1 à 7, **caractérisé en ce que** le dispositif d'actionnement comprend une pompe péristaltique, le volume de fluide à transférer étant ajusté par un déplacement angulaire du rotor de ladite pompe.

9. Procédé selon l'une des revendications 1 à 8, **caractérisé en ce que** le système occlusif est un sphincter urinaire artificiel.

10. Dispositif de détection d'une fuite lente dans un système occlusif hydraulique implantable dans le corps humain ou animal pour occlure un conduit naturel (10), ledit système occlusif comprenant :
- un circuit hydraulique comprenant :
- une manchette occlusive gonflable (9) contenant un volume variable d'un fluide, entourant une partie du conduit naturel (10) à occlure,
- un réservoir (4) contenant un fluide, et
- une liaison fluidique (8) entre la manchette (9) et le réservoir (4),
- un dispositif d'actionnement (2) couplé à un élément mobile (22) dudit circuit hydraulique et adapté pour déplacer ledit élément mobile pour transférer un volume déterminé de fluide du réservoir (4) vers la manchette (9) ou de la manchette vers le réservoir, de sorte à faire varier la compression exercée par ladite manchette (9) sur ledit conduit (10),
- une unité de commande (7) adaptée pour solliciter le dispositif d'actionnement (2) de sorte à exercer une compression déterminée sur le conduit (10),
ledit dispositif de détection comprenant :
- un capteur adapté pour mesurer la pression dans le circuit hydraulique,
- un capteur adapté pour mesurer une sollicitation appliquée au dispositif d'actionnement (2), ladite sollicitation déterminée étant une position de l'élément mobile (22) définissant un volume déterminé de fluide transféré,
- une unité de traitement adaptée pour :
• à partir des données de mesure desdits capteurs, mesurer l'évolution de la pression dans le circuit hydraulique pour une sollicitation déterminée du dispositif d'actionnement (2),
• détecter une fuite lente dans le circuit hydraulique lorsque la pression mesurée dans ledit circuit pour ladite sollicitation déterminée du dispositif d'actionnement (2) remplit un critère déterminé.

11. Système occlusif hydraulique implantable dans le corps humain ou animal pour occlure un conduit naturel (10), comprenant un dispositif adapté pour mettre en oeuvre le procédé de détection d'une fuite lente selon l'une des revendications 1 à 9.

## Patentansprüche

1. Detektionsverfahren eines langsamen Lecks in einem verschließenden hydraulischen System, das in den menschlichen oder tierischen Körper implantierbar ist, um eine natürliche Leitung (10) zu verschließen, wobei das genannte verschließende System umfasst:
- einen hydraulischen Kreislauf:
- eine aufblasbare, verschließende Manschette (9), die ein variables Volumen einer Flüssigkeit enthält und einen Teil des zu verschließenden natürlichen Kanals (10) umgibt,
- einen Tank (4), der eine Flüssigkeit enthält, und;
- eine fluidische Verbindung (8) zwischen der Manschette (9) und dem Tank (4);
- eine Betätigungsvorrichtung (2), die an ein mobiles Element (22) des genannten hydraulischen Kreislaufs gekoppelt und geeignet ist, um das genannte mobile Element zu verschieben, um ein bestimmtes Volumen der Flüssigkeit des Tanks (4) zur Manschette (9) oder der Manschette zum Tank derart zu transferieren, dass die auf die genannte Manschette (9) auf der genannten Leitung (10) ausgeübte Druck verändert wird;
- eine Steuereinheit (7), die zum Ansprechen der Betätigungseinheit (2) derart geeignet ist, dass eine bestimmte Komprimierung auf die Leitung (10) ausgeübt wird,
wobei die Variation der von der Manschette auf die Leitung (10) ausgeübten Komprimierung per Transfer eines einstellbaren Volumens der genannten Flüssigkeit zwischen dem Tank (4) und der Manschette (9) realisiert ist,
wobei das genannte Verfahren umfasst:
- die Messung der Entwicklung des Drucks in dem hydraulischen Kreislauf für ein bestimmtes Ansprechen der Betätigungsvorrichtung (2), wobei das bestimmte Ansprechen in einer Position des mobilen Elements (22) ist, die ein bestimmtes Volumen der transferierten Flüssigkeit definiert,
- die Detektion eines langsamen Lecks in dem hydraulischen Kreislauf, wenn der Druck, der in dem genannten Kreislauf für das genannte bestimmte Ansprechen der Betätigungsvorrichtung (2) ein vorbestimmtes Kriterium erfüllt.

2. Verfahren gemäß Anspruch 1, **dadurch gekennzeichnet, dass** das genannte vorbestimmte Detektionskriterium aus einer der folgenden Bedingungen oder einer Kombination der genannten Bedingungen ausgewählt ist, um ein langsames Leck zu detektieren, das sich durch einen Flüssigkeitsverlust des hydraulischen Kreislaufs manifestiert:
- der Druck im hydraulischen Kreislauf liegt unter einem festen Wert,
- der Druck im hydraulischen Kreislauf liegt unter einem Prozentsatz eines Wertes des ursprünglich für das bestimmte Ansprechen der Betätigungsvorrichtung gemessenen Drucks, und
- ein Wert, der aus einer mathematischen Funktion stammt, die ausgehend von einer Werte-Datenbank des Drucks in dem hydraulischen Kreislauf für das genannte bestimmte Ansprechen der Betätigungsvorrichtung aufgebaut ist, wobei der genannte Druck im Verlauf der Zeit periodisch registriert ist, unter einem bestimmten Wert liegt.

3. Verfahren gemäß einem der Ansprüche 1 oder 2, **dadurch gekennzeichnet, dass** der Tank (4) ein variables Volumen aufweist, wobei das Volumen durch ein lineares Verschieben eines mobilen Elements (22), das durch ein Betätigungsorgan bewegt ist, eingestellt ist.

4. Verfahren gemäß Anspruch 3, **dadurch gekennzeichnet, dass** das bestimmte Ansprechen der Betätigungsvorrichtung (2) eine Position des mobilen Elements (22) im Anschlag derart ist, dass das Volumen des Tanks (4) maximiert wird.

5. Verfahren gemäß einem der Ansprüche 1 bis 4, **dadurch gekennzeichnet, dass** das bestimmte Detektionskriterium erfüllt ist, wenn der Druck, der in dem hydraulischen Kreislauf für das genannte bestimmte Ansprechen gemessen ist, unter einen negativen oder einen Schwellenwert von Null fällt.

6. Verfahren gemäß Anspruch 1, **dadurch gekennzeichnet, dass** das genannte vorbestimmte Detektionskriterium aus einer der folgenden Bedingungen oder einer Kombination der genannten Bedingungen ausgewählt ist, um ein langsames Leck zu detektieren, das sich durch einen flüssigen Eintrag im hydraulischen Kreislauf manifestiert:
- der Druck in dem hydraulischen Kreislauf ist höher als ein fester Wert,
- der Druck im hydraulischen Kreislauf liegt über einem Prozentsatz eines Wertes des ursprünglich für das bestimmte Ansprechen der Betätigungsvorrichtung gemessenen Drucks, und
- ein Wert, der aus einer mathematischen Funktion stammt, die ausgehend von einer Werte-Datenbank des Drucks in dem hydraulischen Kreislauf für das genannte bestimmte Ansprechen der Betätigungsvorrichtung aufgebaut ist, wobei der genannte Druck im Verlauf der Zeit periodisch registriert ist, unter einem bestimmten Wert liegt.

7. Verfahren gemäß einem der Ansprüche 1 bis 6, **dadurch gekennzeichnet, dass** er darüber hinaus das Ausgeben eines Alarms an einen Benutzer umfasst, wenn das Detektionskriterium eines langsamen Lecks erfüllt ist.

8. Verfahren gemäß einem der Ansprüche 1 bis 7, **dadurch gekennzeichnet, dass** die Betätigungsvorrichtung eine peristaltische Pumpe umfasst, wobei das zu transferierende Leckvolumen durch eine winkelförmige Verschiebung des Rotors der genannten Pumpe eingestellt ist.

9. Verfahren gemäß einem der Ansprüche 1 bis 8, **dadurch gekennzeichnet, dass** das verschließende System ein künstlicher Harn-Schließmuskel ist.

10. Detektionsvorrichtung eines langsamen Lecks in einem hydraulischen verschließenden System, das in den menschlichen oder tierischen Körper implantierbar ist, um eine natürliche Leitung (10) zu verschließen, wobei das genannte verschließende System umfasst:
- einen hydraulischen Kreislauf, umfassend:
- eine Manschette (9), die ein variables Volumen einer Flüssigkeit enthält und einen Teil des zu verschließenden natürlichen Kanals (10) umgibt,
- einen Tank (4), der eine Flüssigkeit enthält, und;
- eine fluidische Verbindung (9) zwischen der Manschette (9) und dem Tank (4);
- eine Betätigungsvorrichtung (2), die an ein mobiles Element (22) des genannten hydraulischen Kreislaufs gekoppelt und geeignet ist, um das genannte mobile Element zu verschieben, um ein bestimmtes Volumen der Flüssigkeit des Tanks (4) zur Manschette (9) oder von der Manschette zum Tank derart zu verschieben, dass der auf die genannte Manschette (8) auf der genannten Leitung (10) ausgeübte Druck verändert wird;
- eine Steuereinheit (7), die zum Ansprechen der Betätigungseinheit (2) derart geeignet ist, dass eine bestimmte Komprimierung auf die Leitung (10) ausgeübt wird,
wobei die genannte Detektionsvorrichtung umfasst:
- einen geeigneten Sensor zum Messen des Drucks in dem hydraulischen Kreislauf,
- einen geeigneten Sensor zum Messen eines auf die Betätigungsvorrichtung (2) angewandten Ansprechens, wobei das bestimmte Ansprechen eine Position des mobilen Elements (22) ist, das ein bestimmtes Volumen der transferierten Flüssigkeit definiert,
- eine Verarbeitungseinheit, die geeignet ist, um:
• ausgehend von den Messdaten der genannten Sensoren die Entwicklung des Drucks im hydraulischen Kreislauf für ein bestimmtes Ansprechen der Betätigungsvorrichtung (2) zu messen,
• ein langsames Leck im hydraulischen Kreislauf zu detektieren, wenn der Druck, der in dem genannten Kreislauf für das genannte bestimmte Ansprechen der Betätigungsvorrichtung (2) gemessen ist, ein bestimmtes Kriterium erfüllt.

11. Verschließendes, hydraulisches System, das in den menschlichen oder tierischen Körper implantierbar ist, um einen natürlichen Kanal (10) zu verschließen, umfassend eine Vorrichtung, die geeignet ist, um das Detektionsverfahren eines langsamen Lecks gemäß einem der Ansprüche 1 bis 9 umzusetzen.

## Claims

1. A method for detection of a slow leak in a hydraulic occlusive system implantable in an animal or human body to occlude a natural conduit (10), said occlusive system comprising:
- a hydraulic circuit comprising:
- an inflatable occlusive cuff (9) containing a variable volume of fluid, surrounding a part of the natural conduit (10) to be occluded,
- a reservoir (4) containing fluid, and
- a fluidic connection (8) between the cuff (9) and the reservoir (4),
- an activation device (2) coupled to a mobile element (22) of said hydraulic circuit and adapted to move said mobile element to transfer a determined volume of fluid from the reservoir (4) to the cuff (9) or from the cuff to the reservoir, so as to vary the compression exerted by said cuff (9) on said conduit (10),
- a control unit (7) adapted to urge the activation device (2) so as to exert determined compression on the conduit (10),
variation of the compression exerted by the cuff on the conduit (10) being created by transfer of an adjustable volume of said fluid between the reservoir (4) and the cuff (9), said method comprising:
- measuring the evolution of the pressure in the hydraulic circuit for a determined strain of the activation device (2), said determined strain being a position of the mobile element (22) defining a determined volume of transferred fluid,
- detecting a slow leak in the hydraulic circuit when the pressure measured in said circuit for said determined strain of the activation device (2) fulfils a predetermined criterion.

2. The method according to claim 1, **characterized in that** to detect a slow leak manifesting as a loss of fluid of the hydraulic circuit, said predetermined detection criterion is selected from one of the following conditions or a combination of said conditions:
- the pressure in the hydraulic circuit is less than a fixed value,
- the pressure in the hydraulic circuit is less than a percentage of a value of the pressure measured initially for said determined strain of the activation device, and
- a value obtained from a mathematical function constructed from a database of values of the pressure in the hydraulic circuit for said determined strain of the activation device, said pressure being recorded periodically over time, is less than a determined value.

3. The method according to one of claims 1 or 2, **characterized in that** the reservoir (4) has a variable volume, the volume being adjusted by linear displacement of a mobile element (22) moved by an actuator.

4. The method according to claim 3, **characterized in that** the determined strain of the activation device (2) is a stop position of the mobile element (22) so as to maximise the volume of the reservoir (4).

5. The method according to one of claims 1 to 4, **characterized in that** the determined detection criterion is fulfilled when the pressure measured in the hydraulic circuit for said determined strain becomes less than a zero or negative threshold value.

6. The method according to claim 1, **characterized in that** to detect a slow leak resulting in an addition of fluid into the hydraulic circuit, said predetermined detection criterion is selected from one of the following conditions or a combination of said conditions:
- the pressure in the hydraulic circuit is greater than a fixed value,
- the pressure in the hydraulic circuit is greater than a percentage of a value of the pressure measured initially for said determined strain of the activation device, and
- a value obtained from a mathematical function constructed from a database of values of said pressure for said determined strain of the activation device, said pressure being recorded periodically over time, is greater than a determined value.

7. The method according to one of claims 1 to 6, **characterized in that** it further comprises sending an alarm to a user if the detection criterion of a slow leak is fulfilled.

8. The method according to one of claims 1 to 7, **characterized in that** the activation device comprises a peristaltic pump, the volume of fluid to be transferred being adjusted by angular displacement of the rotor of said pump.

9. The method according to one of claims 1 to 8, **characterized in that** the occlusive system is an artificial urinary sphincter.

10. A device for detection of a slow leak in a hydraulic occlusive system implantable in an animal or human body to occlude a natural conduit (10), said occlusive system comprising:
- a hydraulic circuit comprising:
- an inflatable occlusive cuff (9) containing a variable volume of fluid, surrounding a part of the natural conduit (10) to be occluded,
- a reservoir (4) containing fluid, and
- a fluidic connection (8) between the cuff (9) and the reservoir (4),
- an activation device (2) coupled to a mobile element (22) of said hydraulic circuit and adapted to move said mobile element to transfer a determined volume of fluid from the reservoir (4) to the cuff (9) or from the cuff to the reservoir, so as to vary the compression exerted by said cuff (9) on said conduit (10),
- a control unit (7) adapted to urge the activation device (2) so as to exert determined compression on the conduit (10),
said device for detection comprising:
- a sensor adapted to measure the pressure in the hydraulic circuit,
- a sensor adapted to measure strain applied to the activation device (2), said determined strain being a position of the mobile element (22) defining a determined volume of transferred fluid,
- a processing unit adapted to:
• from measuring data of said sensors, measure the evolution of the pressure in the hydraulic circuit for a determined strain of the activation device (2),
• detect a slow leak in the hydraulic circuit when the pressure measured in said circuit for said determined strain of the activation device (2) fulfils a determined criterion.

11. A hydraulic occlusive system implantable in an animal or human body to occlude a natural conduit (10), comprising a device adapted to carry out the detection method of a slow leak according to one of claims 1 to 9.
